# FASCICULE DE BREVET EUROPEEN

(11) **EP 1 555 999 B1**
(45) Date de publication et mention de la délivrance du brevet: **29.08.2012**
(21) Numéro de dépôt: 03758186.5
(22) Date de dépôt: 25.07.2003
(51) Int. Cl.: A61K 8/97, A61Q 19/00

(54) **Utilisation cosmétique du kombucha pour lutter contre le vieillissement de la peau**
Kosmetische Verwendung von Kombucha zur Behandlung von Hautalterung
Cosmetical use of kombucha for treating skin aging

(30) Priorité: 30.07.2002 FR 0209710
(43) Date de publication de la demande: 27.07.2005
(73) Titulaire: Sederma S.A.S., 78612 Le Perray-en-Yvelines Cedex (FR)
(72) Inventeur: LINTNER, Karl, F-75015 Paris (FR)
(86) Numéro de dépôt international: PCT/FR2003/002368
(87) Numéro de publication internationale: WO 2004/012650

(56) Documents cités:
- WO-A-99/62478
- DUFRESNE, C. ET AL: "Tea, Kombucha, and health: a review" FOOD RESEARCH INTERNATIONAL (2000), 33(6), 409-421 , 2000, XP002270260
- FONTANA J D ET AL: "ACETOBACTER CELLULOSE PELLICLE AS A TEMPORARY SKIN SUBSTITUTE" APPLIED BIOCHEMISTRY AND BIOTECHNOLOGY, vol. 24-25, no. SPRING-SUMMER, 1990, pages 253-264, XP009025594 ELEVENTH SYMPOSIUM ON BIOTECHNOLOGY FOR FUELS AND CHEMICALS, COLORADO SPRINGS, COLORADO, USA, MAY 8- ISSN: 0273-2289
- SADJADI J: "Cutaneous anthrax associated with the Kombucha 'mushroom' in Iran [8]" JOURNAL OF THE AMERICAN MEDICAL ASSOCIATION 11 NOV 1998 UNITED STATES, vol. 280, no. 18, 11 novembre 1998 (1998-11-11), pages 1567-1568, XP009025610 ISSN: 0098-7484
- STEINKRAUS K H ET AL: "INVESTIGATIONS INTO THE ANTIBIOTIC ACTIVITY OF TEA FUNGUS/KOMBUCHA BEVERAGE" ACTA BIOTECHNOLOGICA, AKADEMIE VERLAG, BERLIN, DE, vol. 16, no. 2/3, 1996, pages 199-205, XP002071797 ISSN: 0138-4988
- "Skin external preparation as lotion and base cosmetics, for preventing aging, wrinkles and roughness of skin, contains extract and/or extracted fraction of Pueraria root and Sanguisorba officinalis", DERWENT, XP002236244,

## Description

La présente invention relève du domaine des cosmétiques et concerne l'utilisation du kombucha et des compositions cosmétiques ou dermopharmaceutiques le contenant, seul ou en association, pour tous les soins de la peau, des muqueuses et des phanères, et en particulier pour prévenir les signes du vieillissement endogène et/ou exogène.

La peau, les muqueuses, et les phanères sont des éléments fragiles qui méritent les plus grands égards. En effet, leur équilibre est perturbé en permanence par des agressions extérieures, soleil, vent, froid. Le vieillissement de la peau est un des premiers signes extérieurs du vieillissement. Il résulte d'un certain nombre d'altérations qui surviennent spontanément au cours du temps mais qui peuvent également être induites par des facteurs externes (radiations solaires, fumée de tabac, consommation excessive d'alcool).

Les signes du vieillissement se traduisent cliniquement par l'apparition de rides et ridules, par un relâchement des tissus cutanés et sous-cutanés qui se traduit par une texture de peau atone, un relâchement du micro-relief cutané, une fermeté cutanée diminuée, une perte d'élasticité, et une peau globalement flasque, plus terne et sans éclat.

Le vieillissement se traduit aussi par une diminution du nombre, de l'épaisseur et de la croissance des cheveux, une baisse des sécrétions (sébacées et sudorales), et une altération de la structure et de la croissance des ongles. Sur les zones de la peau qui ont été exposées au soleil tout au long de la vie - essentiellement le visage, le décolleté, les mains et les avant bras - on observe souvent des tâches d'hyper- ou hypopigmentation.

Certains de ces signes sont plus particulièrement liés au vieillissement intrinsèque ou physiologique, c'est-à-dire au vieillissement lié à l'âge, alors que d'autres sont plus spécifique du vieillissement extrinsèque, c'est à dire du vieillissement provoqué d'une manière générale par l'environnement ; il s'agit plus particulièrement du photo-vieillissement dû à l'exposition au soleil, à la lumière ou à tout autre rayonnement.

La présente invention s'intéresse au traitement général du vieillissement de la peau, des muqueuses et des phanères, notamment par l'amélioration de l'éclat du teint, et en particulier par la prévention ou le ralentissement de la glycation, ainsi que par la stimulation de la synthèse des lipides par les adipocytes cutanés.

Il existe deux types de glycolysation du collagène. L'une, enzymatique, naturelle et nécessaire, est une étape de la formation du collagène. L'autre, qui est une glycation aspécifique, est une réaction chimique qui se produit spontanément (sans l'intervention d'un système enzymatique), un peu partout dans l'organisme, entre le glucose et de nombreux constituants biochimiques tels que les protéines à longue durée de vie [THORPE SR, 1996 ; NAGARAJ RH, 1996]. Ces réactions spontanées sont connues sous le nom de réaction de Maillard. Les principales causes physiologiques actuellement connues comme responsable de ce phénomène sont le diabète (du fait du taux anormalement élevé du glucose sanguin) et le vieillissement. Les pathologies qui découlent de la glycation dans le cas du diabète sont notamment la cataracte, des neuropathies diverses, des atteintes rénales, et micro et macroangiographies. Dans le cas du vieillissement, les complications les plus souvent rencontrées atteignent le collagène : avec l'âge, la résistance mécanique (à l'étirement) du collagène augmente. La glycosylation des fibres du collagène et des éléments du tissu conjonctif en général, en créant des réticulations définitives entre les différentes fibres, entraînant leur rigidification, et de là une perte de souplesse et d'élasticité de la peau.

L'âge avançant, les cellules de la peau (et notamment les fibres de collagène qui sont le principal constituant de la matrice extracellulaire) se renouvellent moins bien et moins vite. Le derme s'amincit, mais la couche superficielle s'épaissit par une élimination défectueuse des cellules mortes.

Au vu de ces observations, on en déduit que le ralentissement de la glycation du collagène cutané permettra à la peau de conserver une souplesse et une élasticité bien meilleure, améliorant ainsi son aspect et la sensation de confort.

La description de cette demande de brevet concerne donc un produit ayant un fort pouvoir anti-glycation.

Par ailleurs, on constate qu'une peau éclatante, telle une peau de bébé, est une peau qui réfléchit autrement la lumière. Elle est lisse, avec un micro-relief amélioré, tendue.

Redonner de la matière à la peau, redensifier le derme, est donc également une approche valable pour lutter contre les rides et les ridules, pour aider la peau à retrouver de l'éclat et un aspect plus jeune.

Il n'en est pour s'en convaincre que de constater le nombre croissant de dermatologues qui utilisent des injections intradermiques de collagène, d'acide hyaluronique. Aujourd'hui, il existe une nouvelle technique : le lipofilling, qui consiste en des réinjections de graisse prélevée sur le patient pour boucher les vides tissulaires responsables de rides. Toutes ces méthodes présentent toutefois certains inconvénients plus ou moins importants. Outre le prix de ces interventions, la graisse, le collagène, ou l'acide hyaluronique injectés peuvent toujours être reconnus comme faisant partie du *non-soi* et ainsi provoquer des réactions inflammatoires forcément indésirables. De plus, ce matériel biologique surajouté artificiellement, subira rapidement la dégradation enzymatique normale, ce qui induira des cycles d'injections selon des fréquences de plus en plus rapprochées.

La description de cette demande de brevet concerne également un produit qui permet de mimer la chirurgie esthétique. Il s'agit ainsi donc de faire du « lipofilling » cosmétique en effectuant un remplissage cutané par augmentation de la masse adipocytaire, qui, étant néoformée par l'organisme lui-même, gommera ou réduira l'importance des rides tout en assurant une parfaite tolérance et indolore à l'usager. Le principe de ce lipofilling cosmétique est la stimulation de la synthèse des lipides par les adipocytes cutanés. Ces effets ont été étudiés et démontrés, *in vitro* et *in vivo,* et seront explicités dans les exemples donnés dans cette demande de brevet.

Un des buts est donc de pouvoir proposer un produit qui présente une efficacité contre le vieillissement cutané et capillaire en général et en particulier contre la glycation et la diminution des lipides sous-cutanés, et ce sans effets secondaires notables.

Beaucoup de produits naturels, comme le thé vert, sont déjà utilisés dans les produits cosmétiques et prétendent traiter les rides et les ridules de la peau ou raffermir les tissus cutanés, redonner de l'éclat à la peau. Mais ces compositions ne traitent qu'incomplètement et temporairement ces désordres morphologiques et leur utilisation est souvent non évidente. Le thé vert par exemple a tendance à noircir les produits finis, et son activité est limitée : les propriétés revendiquées pour le thé vert sont la protection anti-radicalaire et l'activité anti-oxydante, voire l'amincissement lorsque l'extrait de thé vert est dosé en caféine.

Or, nous avons découvert de manière surprenante et inattendue que le kombucha, en application topique, possède des activités cosmétiques intéressantes qui permettent de lutter contre les signes du vieillissement de façon plus spécifique, luttant contre la glycation, et les différentes lacunes du métabolisme de la peau, améliorant le micro-relief cutané.

Le kombucha est un thé qui a subit une transformation microbiologique. Cette transformation est une fermentation par un symbiote de levure du genre Saccharomyces qui nidifie au sein d'une matrice de polysaccharides produite par une Bactérie Xylinum. La composition exacte de ce symbiose (en particulier les proportions de chacune des espèces) varie avec les conditions géographiques et climatiques et dépend des sous-espèces locales sauvages de levure et de bactéries ; néanmoins on peut citer, entre autres, sans que cette liste soit limitative : Saccharomyces ludwigii, espèces Saccharomyces apicalutus, Bacterium xylinoides, Bacterium gluconicum, Schizosaccharomyces pombe, Acetobacter ketogenum, espèces Torula, Pichia fermentans et autres levures. Dans la littérature sur le kombucha, ce symbiote de levures et bactéries est aussi appelé « champignon », « champion de longue vie » et de nombreux synonymes.

Cette fermentation est tout à fait originale puisque l'alcool produit par la levure à partir du sucre est transformé par la bactérie en différents acides, glucuronique, lactique, usnique et surtout acétique qui donne son goût acidulé au kombucha avec un pH final compris entre 2,5 et 4. D'autre part, la bactérie Xylinum qualifiée de « bactérie acétique », utilise elle aussi le sucre présent dans le thé et transforme le saccharose en micro-fibrilles de cellulose constituant ainsi la membrane support dans laquelle la levure nidifie et se développe.

Les produits du métabolisme de la levure sont excrétés dans le kombucha et sont constitués de nombreuses vitamines telles que B1, B2, B3 et B12, co-facteurs essentiels à la croissance des bactéries.

Le kombucha, également appelé « thé au champignon de longue vie », est un remède thérapeutique populaire connu depuis longtemps par différents noms [FRANK G., 1999]. C'est une boisson rafraîchissante à la couleur jaune-ambre avec un goût doux-acide de cidre. Son effet est connu et apprécié depuis des générations par de nombreux peuples, surtout dans les pays de l'Asie orientale. Le thé utilisé pour la fabrication de kombucha peut être de toute sorte et de toute origine, il s'agit notamment de *Camellia sinensis* variétés *sinensis* ou *assamica.* Toutes les variétés de thé vert, thé semi-fermenté, thé noir, thé noir fumé, thé jaune, thé sombre, thé blanc, de tisane aux plantes ou aux fruits, d'infusion, peuvent servir de base à la fabrication du kombucha. On utilise préférentiellement toutes les variétés de thé vert, thé semi-fermenté, thé noir, thé noir fumé, thé jaune, thé sombre, thé blanc (tous *Camellia sinsensis)* et plus particulièrement le thé noir.

Parfois il peut y avoir confusion autour du terme thé fermenté. En effet, le thé noir est du thé vert appelé « fermenté », mais cette transformation n'est pas une fermentation à proprement parler, il s'agit d'une oxydation que subit le thé quand sont mis en contact certains substrats par une rupture mécanique des cellules en rompant ou en coupant les feuilles. Par contre, pour obtenir du thé vert, immédiatement après la cueillette, pour éviter l'oxydation des tanins et préserver la chlorophylle, les feuilles doivent être mises faiblement sous vapeur, ensuite les feuilles sont roulées et séchées.

Pour la fabrication du kombucha le thé noir est le meilleur bouillon de culture : il conduit à la plus grande concentration d'acide lactique et gluconique, offre les meilleures conditions à la croissance du « champignon », et est riche en purines.

Le « champignon », ainsi que la recette de la fabrication du kombucha se transmettent de génération en génération. Le procédé de fabrication du kombucha est présenté en exemple pour illustrer la présente invention.

Le terme « kombucha » dans la présente invention inclut les synonymes tels que comboucha, cajnyj kvas (russe), Combuchagetränk (allemand), Kargasoktee (allemand), komboecha-drank (néerlandais), Kombuchakwaß (allemand), tea-beer et tea-cider (anglais), etc ...

De la même façon, le symbiote, « champignon de longue vie » est aussi appelé, notamment : cajnyj grib (russe), champignon japonais ou chinois, champignon miracle, comboucha, combucha (japonais), fungojapon, fungus japonicus (désignation pharmaceutique), funko cinese (italien), ganoderma japonicum, japanischer Teepilz (allemand), kombucha, ling zhi (chinois), mandschurischer Schwamm, russische Blume, tea fungus kombucha, Wolgaqualle (allemand), Zauberpilz, etc ....

Le kombucha produit, outre de nombreuses substances à effet antibiotique difficilement définissables, avant tout de l'acide glucuronique, les vitamines B1, B2, B3, B6 et B12 ainsi que l'acide folique, et de l'acide lactique D (+).

Il existe de nombreuses expériences thérapeutiques sur le kombucha. Dans les pays asiatiques et en Russie, le kombucha est utilisé depuis des siècles comme moyen thérapeutique naturel avec grand succès. Outre l'utilisation comme boisson rafraîchissante, on cite un grand nombre de maladies combattues avec succès par le kombucha qui vont de l'indisposition la plus futile à la maladie la plus grave [Hagers Handbuch für die pharmaceutische Praxis, 1973, pp 254-256]. Dufresnes et al. [Food Research International 33 (2000) pp409-421] décrivent des produits comprenant du kombucha.

L'objet de cette demande de brevet réside dans la découverte, et dans la démonstration, que le kombucha, convenablement préparé et utilisé, se révèle être une nouvelle solution pour combattre les signes du vieillissement exposés précédemment.

Lors du développement du projet qui a abouti à cette demande de brevet, nous avons trouvé que les activités bénéfiques du kombucha sur les applications mentionnées ci-dessus s'expliquent par sa composition. Le kombucha, est en effet riche en vitamines, acides aminés, sucres, et en épigallocatéchine gallate (EGCG).

De ce fait, l'application topique du kombucha relance l'activité de l'ensemble des types cellulaires cutanés (kératinocytes, mélanocytes, fibroblastes et adipocytes). Elle relance aussi l'activité immunitaire par la stimulation des cellules de Langherans, et l'activité des adipocytes jusqu'à la lipogénèse. Le kombucha peut donc être considéré comme un agent restructurant contre le vieillissement, par son activité nutritive.

En effet, de manière surprenante, nous avons observé et démontré que le kombucha, par application topique, est efficace contre la glycation. Il est donc capable d'entretenir et de redonner de l'élasticité et de la souplesse à la peau vieillie de manière intrinsèque et/ou extrinsèque.

Nous avons aussi observé et démontré que le kombucha, par application topique, stimule la synthèse des lipides par les adipocytes cutanés. Il est donc capable de prévenir et traiter les rides et les ridules de la peau, de raffermir les tissus cutanés du visage, des hanches, cuisses, de redonner de l'éclat à la peau par l'amélioration du micro-relief cutané ...

Nous avons également observé et démontré que le kombucha, par application topique, permet d'effectuer un « lipofilling » cosmétique.

Le kombucha, est aussi capable d'assurer une meilleure irrigation cutanée, de régulariser l'activité des mélanocytes.

Le kombucha, utilisé dans les compositions cosmétiques ou dermopharmaceutiques objets de cette invention, peut être fabriqué à partir de toute source de thé, tisane, infusion, par fermentation à l'aide de toute combinaison de champignons, levures et bactéries, en particulier celles citées plus haut. L'application topique de kombucha n'a toutefois encore jamais été décrite. La présente invention concerne donc les compositions cosmétiques ou dermopharmaceutiques contenant du kombucha.

Les compositions faisant contiennent un milieu cosmétiquement ou dermatologiquement acceptable, c'est-à-dire compatible avec les tissus cutanés. Ainsi, la composition peut être appliquée sur tout le corps humain, y compris les muqueuses et les phanères.

Le kombucha est utilisé à titre d'exemple en une quantité allant de 0,001% (p/p) à 100% (p/p) du poids total de la composition, préférentiellement entre 0,01% (p/p) et 20% (p/p).

Les compositions sont par exemple des lotions, des laits ou des crèmes émollientes, des laits ou des crèmes pour les soins de la peau ou des cheveux, des crèmes, des lotions ou des laits démaquillants, des bases de fond de teint, des lotions, des laits ou des crèmes solaires, des lotions, des laits ou des crèmes de bronzage artificiel, des crèmes ou des mousses de rasage, des lotions après-rasage, des shampoings, des rouges à lèvres, des mascaras ou des vernis à ongles.

Ces compositions peuvent également se présenter sous la forme de bâtons pour les lèvres destinés soit à les colorer, soit à éviter les gerçures, ou de produits de maquillage pour les yeux ou des fards et fonds de teint pour le visage.

Lorsque les compositions se présentent sous la forme d'émulsions de type eau-dans-l'huile ou huile-dans-l'eau, la phase grasse est essentiellement constituée d'un mélange de corps gras d'extraction ou de synthèse, avec au moins une huile, et éventuellement un autre corps gras. La phase grasse des émulsions peut constituer de 5 à 60% du poids total de l'émulsion.

La phase aqueuse desdites émulsions constitue de préférence de 30 à 85% du poids total de l'émulsion. La proportion de l'agent émulsionnant peut être comprise entre 1 et 20%, et de préférence entre 2 et 12% du poids total de l'émulsion. Lorsque les compositions se présentent sous forme de lotions huileuses, oléoalcooliques ou hydroalcooliques, elles peuvent constituer, par exemple, des lotions antisolaires contenant un filtre absorbant les rayons UV, des lotions adoucissantes pour la peau ; les lotions huileuses peuvent en outre constituer des huiles moussantes contenant un tensioactif oléosoluble, des huiles pour le bain, etc ...

Parmi les principaux adjuvants pouvant être présents dans les compositions , on peut citer les solvants organiques ou hydroglycoliques, y compris le MP-diol et les polyglycérines, les corps gras d'extraction ou de synthèse, les épaississants ioniques ou non ioniques, les adoucissants, opacifiants, stabilisants, émollients, les silicones, α- ou ß-hydroxyacides, les agents anti-mousse, les agents hydratants, les vitamines, parfums, conservateurs, séquestrants, colorants, les polymères gélifiants et viscosants, les tensioactifs et émulsifiants, les autres principes actifs hydro- ou liposolubles, les extraits végétaux, extraits tissulaires, extraits marins, filtres solaires, les antioxydants.

Les mono-ou poly-alcools plus particulièrement préférés sont choisis parmi l'éthanol, l'isopropanol, le propylène-glycol, le glycérol et le sorbitol.

A titre de corps gras, parmi les huiles minérales, on peut citer l'huile de vaseline ; parmi les huiles animales, les huiles de baleine, de requin, de phoque, de menhaden, de foie de flétan, de morue, de thon, de tortue, de pied de boeuf, de pied de cheval, de pied de mouton, de vison, de loutre, de marmotte, etc ... ; parmi les huiles végétales, les huiles d'amande, de germes de blé, de jojoba, de sésame, de tournesol, de palme, de noix, de karité, de shoréa, de macadamia, de pépins de cassis et similaires.

Parmi les esters d'acides gras, on peut utiliser des esters d'acide en C₁₂ à C₂₂ saturés ou insaturés et d'alcools inférieurs comme l'isopropanol ou le glycérol ou d'alcools gras en C₈ à C₂₂, linéaires ou ramifiés, saturés ou insaturés ou encore d'alcanediols-1,2 en C₁₀-C₂₂.

On peut également citer comme corps gras, la vaseline, la paraffine, la lanoline, la lanoline hydrogénée, le suif, la lanoline acétylée, les huiles de silicone.

Parmi les cires, on peut citer la cire de Sipol, la cire de lanoline, la cire d'abeille, la cire de Candelila, la cire monocristalline, la cire de Carnauba, le spermaceti, le beurre de cacao, le beurre de karité, les cires de silicone, les huiles hydrogénées concrètes à 25°C, les sucroglycérides, les oléates, myristates, linoléates et stéarates de calcium, magnésium et aluminium.

Parmi les alcools gras, on peut citer les alcools laurique, cétylique, myristique, stéarique, palmitique, oléique, et les alcools de GUERBET comme le 2-décyltétradécanol ou le 2-hexyldécanol. A titre d'émulsionnants, parmi les alcools gras polyoxyéthylénés, on peut citer les alcools laurique, cétylique, stéarylique et oléique comportant de 2 à 20 moles d'oxyde d'éthylène et parmi les alcoyléthers de glycérol, les alcools en C₁₂-C₁₈ comportant de 2 à 10 moles de glycérol. Il peut être aussi utile d'utiliser des épaississants tels que les dérivés de cellulose, les dérivés de d'acide polyacrylique, les gommes de guar ou de caroube ou la gomme de xanthane.

Les compositions peuvent inclure des autre ingrédients variés et additionnels, conventionnels ou non. Bien sûr la décision d'inclure un ingrédient et le choix d'un actif spécifique et des ingrédients additionnels dépend de l'application spécifique et de la formulation du produit. La ligne de démarcation entre un ingrédient "actif" et un ingrédient "additionnel" est donc artificielle et dépend de l'application spécifique et du type de produit. Une substance qui est un ingrédient "actif" dans une application ou un produit peut être un ingrédient "additionnel" ou "fonctionnel" dans une autre, et *vice versa.*

Les compositions peuvent donc inclure un ou plusieurs ingrédients actifs, qui apporteront un certain avantage à l'objet de l'application de la composition, par exemple la peau ou les cheveux. Ces ingrédients actifs peuvent inclure une ou plusieurs substances comme des agents de nettoyage, agents de soin capillaire, agent de soin pour la peau, agent de coiffage, agent antipelliculaire, agent de repousse de cheveux, parfums, écrans solaires, pigments, hydratants, agents filmogènes, colorants capillaires, maquillages, détergents, agents épaississants, émulsifiants, agents antiseptiques, actifs déodorants et surfactants, propulseurs.

Le choix de l'actif ou des actifs dépend de la nature du produit cosmétique ou de soin à formuler. Par exemple, les filtres solaires peuvent être utilisés dans des lotions anti-solaires, dans des shampooings, dans des lotions de soin capillaire, et ainsi de suite. Pour chaque type d'actif, un ou plusieurs ingrédients peuvent être présents. De même, plus d'un type d'actif peut être présent dans une composition.

Le CTFA Cosmetic Ingredient Handbook, Ninth Edition (2002) liste beaucoup des ingrédients cosmétiques habituellement utilisés dans l'industrie cosmétique, qui conviennent pour être utilisés dans les compositions selon la présente description. Des exemples de classes d'ingrédients incluent : les abrasifs, les absorbants, les huiles essentielles, les astringents, les agents anti-agglomérants, les agents anti-mousses, les agents anti-microbiens, les antioxidants, les liants, les additifs bilologiques, les tampons, les agents de volume, les agents chelatants, les additifs chimiques, les colorants, les astringents cosmétiques, les biocides cosmétiques, les dénaturants, les astringents de médicaments, les analgésiques externes, les agents filmogènes, les agents opacifiants, les ajusteurs de pH, les propellants, les agents de réduction, les séquestrants, les agents de soin de la peau (par exemple les humectants), les agents de traitement de la peau, les épaississants, mais aussi les actifs pris parmi le groupe consistants des actifs de desquamation, les actifs anti-acné, la vitamine C et ses dérivés, les vitamines B1 à B12 leurs dérivés, la vitamine E et ses dérivés, la vitamine H, la vitamine K, la vitamine A et les retinoïdes, les peptides, les hydroxy acides, les anti-oxidants, les piègeurs de radicaux, les chélatants, les actifs anti-inflammatoires, les actifs bronzants, les actifs éclaircissants de la peau, actifs anti-cellulite, les actifs anti-microbiens, les actifs anti-rides, les actifs stimulant la lipolyse, les actifs stimulant la lipogénèse, les agents anti-stress, les inhibiteurs de la protéolyse, en particulier les inhibiteurs de la MMP, les enzymes, les ceramides et leurs analogues, les anti-irritants et les actifs adoucissant la peau, les actifs anti-pollution, les actifs cicatrisants, les hydratants, les émollients, les actifs de protection anti-solaire, les écrants solaires et filtres UV, les actifs raffermissants, les liposomes.

En particulier, les ingrédients de soin capillaires qui peuvent être combinés dans les compositions selon la présente description peuvent être trouvés sur www.sederma.fr, ou dans les paragraphes qui suivent.

Les compositions peuvent également contenir une quantité suffisante d'actifs anti-acné. Des exemples d'actifs anti-acné incluent la composition appelée ac.net® (commercialisé par SEDERMA, France) et ses composants individuels (acide nordihydroguaiarétique, acide oléanolique), et aussi le résorcinol, le soufre, l'acide salicylique, le peroxyde de benzoyle, l'érythromycine, le zinc, etc. D'autres exemples d'actifs anti-acné sont décrits en détails dans le brevet U.S. Pat. No. 5 607 980, délivré à McAtee et al., le 4 mars 1997.

Les compositions peuvent également contenir une quantité suffisante d'un ou plusieurs actifs anti-rides. Des exemples d'actifs anti-rides convenant à l'utilisation dans les compositions incluent les alpha-hydroxy acides comme l'acide lactique et placide glycolique ou les béta-hydroxy acides comme l'acide salicylique et ses dérivés, les vitamines, en particulier la vitamine B3 et tous les rétinoïdes. Les isoflavones et les phytostérols conviennent aussi particulièrement.

Des peptides, et notamment des di-, tri-, tetra-, et pentapeptides et leurs dérivés peuvent être inclus dans les compositions en quantité suffisante. Les peptides peuvent être naturels ou synthétiques. Les dipeptides peuvent être, sans être limités à cette liste, Tyr-Arg, Val-Trp, Asn-Phe, Asp-Phe, beta-Ala-His (Carnosine), N-palmitoyl-beta-Ala-His, Tyr-Arg-hexadecylester, et leurs dérivés. Les tripeptides incluent Gly-His-Lys, Arg-Lys-Arg, His-Gly-Gly, Lys-Phe-Lys, Lys-Phe-Lys et leurs analogues de substitution conservative, Gly-His-Lys, Gly-Lys-His, Arg-Lys-Arg-NH₂, et leurs dérivés. Les tetrapeptides incluent Gly-Gln-Pro-Arg (Rigin), Thr-Lys-Pro-Arg (Tuftsin) Lys-Asn-Pro-Tyr, Lys-Asn-Gly-Tyr, Lys-Asn-(D-Pro)-Tyr, Lys-Asn-Pro-Phe, (D-Lys)-Asn-Pro-Tyr, Lys-Gln-Pro-Tyr, Gly-Asn-Pro-(D-Arg), Gly-Asn-Pro-Tyr, (D-Lys)-Asn-Gly-Tyr, (D-Lys)-Gln-Pro-Tyr and (D-Lys)-Asn-Pro-Phe et leurs dérivés et analogues par substitution conservative. Les pentapeptides et hexapeptides peuvent être, sans être limités à cette liste, Lys-Thr-Thr-Lys-Ser, Tyr-Gly-Gly-Phe-X avec X = Met or Leu ou des mélanges, Val-Gly-Val-Ala-Pro-Gly et leurs dérivés. Ces peptides seront utilisés sous leurs formes libres ou N-acylées. En particulier, un dipeptide préféré est le N-Ac-Tyr-Arg-hexadecylester (CALMOSENSINE® de SEDERMA, France). Un tripeptide préféré est le N-palmitoyl-Gly-His-Lys (BIOPEPTIDE CL de SEDERMA, France), le Peptide CK (Arg-Lys-Arg) et la Lipospondine (N-elaidoyl-Lys-Phe-Lys) et ses analogues de substitution conservative, le Peptide CK+ (N-Ac-Arg-Lys-Arg-NH₂). Un tetrapeptide préféré est le N-palmitoyl-Gly-Gln-Pro-Arg et un pentapeptide préféré est le N-Pal-Lys-Thr-Thr-Lys-Ser, disponible sous le nom MATRIXYL® de SEDERMA, France.

Les compositions peuvent inclure des antioxydants et/ou des piégeurs de radicaux pour protéger la peau des dommages causés par une exposition aux UV. Des anti-oxydants/piégeurs de radicaux comme l'acide ascorbique (vitamine C) et ses sels, les esters ascorbiques des acides gras, les dérivés de l'acide ascorbique (par exmple magnesium ascorbyl phosphate, sodium ascorbyl phosphate, ascorbyl sorbate), le tocopherol (vitamine E), le sorbate de tocopherol, l'acétate de tocopherol, les autres esters du tocopherol, les acides benzoïques hydroxy dutylés et leurs sels, 6-hydroxy acide (disponible sous le nom TROLOX®), l'acide gallique et ses alkyls esters, et notamment le propyl gallate, l'acide urique et ses sels et ses alkyl esters, l'acide sorbique et ses sels, l'acide lipoïque, les amines (par exemple la N, N-diéthylhydroxylamine, l'aminoguanidine), les composés sulfhydryles (par exemple glutathion), l'acide dihydroxy fumarique et ses sels, la lysine pidolate, l'arginine pidolate, l'acide nordihydroguaiaretique, les bioflavonoides, le curcumin, la lysine, la methionine, la proline, la superoxide dismutase, les Extremozymes comme celle proposée sous le nom VENUCEANE® (commecialisée par SEDERMA, France), la silymarine, les extraits de thé, les extraits de peau/grains de raisin, la mélanine, et les extraits de romarin peuvent être utilisés.

Les flavonoïdes sont les flavanones choisies parmi les flavanones non substituées, les flavanones monosubstitutées, et leurs mélanges ; les chalcones sélectionnées parmi les chalcones non- substituées, les chalcones mono-substituées, les chalcones di-substituées, les chalcones tri-substituées, et leurs mélanges ; les flavones choisies parmi les flavones non-substituées, les flavones mono-substituées, les flavones di-substituées, et leurs mélanges ; une ou plusieurs isoflavones ; les coumarines choisies parmi les coumarines non-substituées, les coumarines mono-substituées, les coumarines di-substituées, et leurs mélanges ; les chromones choisies parmi les chromones non-substituées, les chromones mono-substituées, les chromones di-substituées, et leurs mélanges ; un ou plusieurs dicoumarols ; une ou plusieurs chromanones ; un ou plusieurs chromanols ; leurs isomères (par exemple les isomères cis/trans) ; et leurs mélanges.

D'autres exemples de flavonoïdes peuvent être trouvés dans la demande PCT No. WO 00/62743 déposée par Larry R. Robinson et al. le 19 avril, 2000, publiée le 26 Octobre, 2000. Ils peuvent être obtenus comme extraits de sources naturelles (plantes, algues), comme produits d'hémisynthèse ou de synthèse. Des mélanges des composés flavonoïdes ci-dessus peuvent aussi être utilisés.

Les compositions peuvent contenir un agent éclaircissant de la peau. Les agents éclaircissants de la peau incluent l'acide kojique, l'arbutine, l'acide ascorbique et ses dérivés (notamment magnesium ascorbyl phosphate ou sodium ascorbyl phosphate), et les extraits (notamment extrait de citrus unshiu, les dérivés de la noraporphine, les extraits de busserole et mitracarpus, respectivement disponibles comme MELASLOW®, LUMISKIN® et ETIOLINE® de SEDERMA, France).

Les agents anti-inflammatoires peuvent être incorporés dans des compositions selon la présente invention, comme des extraits naturels de plantes, fungi, algues. Par exemple, l'acide ursolique, l'acide nordihydroguaiaretique, l'extrait de kava-kava, l'xtrait de bacopa monnieri (BACOCALMINE® de SEDERMA, France), la cire de candelilla, le bisabolol, l'aloe vera, les stérols de plantes, la camomille, l'extrait de trèfle rouge (commecrialisé sous le nom STEROCARE® de SEDERMA, France), et les extraits de sea whip, peuvent être utilisés. D'autres agents anti-inflammatoires utiles incluent les composés de la famille de la réglisse (du genre/espèce Glycvrrhiza glabra), incluant l'acide glycyrrhétique, l'acide glycyrrhizique, et leurs dérivés (notamment leurs sels et esters).

Lorsque les compositions sont des dispersions, il peut s'agir de dispersions de lécithine dans l'eau en présence de tensioactif ou encore de dispersions aqueuses de sphérules lipidiques, constituées de couches moléculaires organisées enfermant une phase aqueuse encapsulée. On peut citer, à cet effet, comme composés lipidiques, les alcools et diols à longue chaîne, les stérols tels que le cholestérol, les phospholipides, les cholestéryl sulfate et phosphate, les amines à longue chaîne et leurs dérivés d'ammonium quaternaire, les dihydroxyalkylamines, les amines grasses polyoxyéthylénées, les esters d'aminoalcools à longue chaîne, leurs sels et dérivés d'ammonium quaternaire, les esters phosphoriques d'alcools gras tels que le dicétylphosphate acide ou son sel de sodium, les alkylsulfates tels que le cétylsulfate de sodium, les acides gras sous forme de sels ou encore les lipides du type de ceux décrits dans les brevets français n° 2 315 991, 1 477 048 et 2 091 516 ou dans les demandes de brevet international WO 83/01 571 et WO 92/08 685.

On peut par exemple utiliser comme autres lipides, des lipides comportant une chaîne lipophile longue contenant 12 à 30 atomes de carbone, saturée ou insaturée, ramifiée ou linéaire, par exemple une chaîne oléique, lanolique, tétradécylique, hexadécylique, isostéarylique, laurique ou alcoylphénylique. Le groupement hydrophile de ces lipides peut être un groupement ionique ou non-ionique. A titre de groupements non-ioniques, on peut citer des groupements dérivés de polyéthylèneglycol. On peut aussi utiliser avantageusement comme lipides formant la phase lamellaire, des éthers de polyglycérol tel que ceux décrits dans les brevets français n°1 477 048 , 2 091 516, 2 465 780 et 2 482 128.

A titre de groupement ionique, on peut avantageusement utiliser un groupement dérivé d'un composé amphotère, anionique ou cationique.

D'autres lipides décrits dans la demande de brevet international WO 83/01 571 comme pouvant être utilisés pour la formation de vésicules sont les glycolipides comme le lactosylcéramide, le galactocérébroside, les gangliosides et le trihexosylcéramide, ainsi que les phospholipides tels que le phosphatidylglycérol et le phosphatidylinositol.

Les substances actives peuvent être des substances ayant un intérêt pharmaceutique, alimentaire ou ayant une activité cosmétique. Lorsqu'elles sont hydrosolubles, elles peuvent être dissoutes de façon homogène ou elles sont dans la phase aqueuse encapsulée à l'intérieur des vésicules. Les substances hydrosolubles ayant une activité cosmétique et/ou pharmaceutique peuvent être des produits destinés aux soins ou aux traitements de la peau et du cheveu tels que par exemple des humectants comme la glycérine, le sorbitol, le pentaérythritol, l'acide pyrrolidone carboxylique et ses sels ; des agents de brunissage artificiel tels que la dihydroxyacétone, l'érythrulose, le glycéraldéhyde, les γ-dialdéhydes tels que l'aldéhyde tartique, ces composés étant éventuellement associés à des colorants; des filtres solaires hydrosolubles ; des antiperspirants, des déodorants, des astringents, des produits rafraîchissants, toniques, cicatrisants, kératolytiques, dépilatoires, des eaux parfumées ; des extraits de tissus végétaux, tels que les polysaccharides ; des colorants hydrosolubles ; des agents antipelliculaires ; des agents antiséborrhéiques, des oxydants tels que des agents de décoloration comme l'eau oxygénée ; des réducteurs tels que l'acide thioglycolique et ses sels.

On peut citer également les vitamines, les hormones, les enzymes, telles que la superoxyde dismutase, les vaccins, les anti-inflammatoires tels que l'hydrocortisone, les antibiotiques, les bactéricides, les agents cytotoxiques ou anti-tumoraux.

Lorsque les substances actives sont liposolubles, elles peuvent se trouver incorporées dans les feuillets des vésicules. Elles peuvent être choisies dans le groupe formé par les filtres solaires liposolubles, les substances destinées à améliorer l'état des peaux sèches ou séniles, les tocophérols, les vitamines E, F ou A et leurs esters, l'acide rétinoïque, les antioxydants, les acides gras essentiels, l'acide glycyrrhétinique, les kératolytiques et les caroténoïdes.

Le kombucha peut être utilisé dans les compositions cosmétiques soit en ajout individuel, soit en tant que pré-mélange dans un excipient convenable, et utilisé sous forme de solution, de dispersion, d'émulsion, de pâte ou de poudre. Il peut être véhiculé par des vecteurs cosmétiques tels que les macro-, micro- ou nanocapsules, des liposomes ou des chylomicrons, les macro-, micro- ou nanoparticules ou microéponges. Il peut être également adsorbé sur des polymères organiques poudreux, les talcs, bentonites et autres supports minéraux.

Il peut être utilisé dans une forme quelconque, ou sous une forme liée ou incorporée ou absorbée ou adsorbée à de macro-, micro- et nanoparticules, de macro-, micro- et nanocapsules, pour le traitement des textiles, fibres synthétiques ou naturelles, laines et tous matériaux susceptibles d'être utilisés pour réaliser des vêtements et sous-vêtements de jour ou de nuit destinés à être en contact avec la peau tel que les collants, sous-vêtements, mouchoirs, lingettes, pour exercer leur effet cosmétique via ce contact textile/peau et permettre une délivrance topique continue.

La description concerne également l'utilisation du kombucha et l'utilisation des compositions cosmétiques et dermopharmaceutiques le contenant, seul ou en association, comme ou pour la fabrication de compositions cosmétiques ou dermopharmaceutiques pour tous les soins de la peau, des muqueuses, et des phanères, notamment pour prévenir les signes du vieillissement endogène et/ou exogène, et en particulier pour redonner souplesse et élasticité à la peau, améliorer son aspect et la sensation de confort, effectuer un « lipofilling » cosmétique, raffermir la peau du visage, des hanches, des cuisses, tonifier la texture de la peau, redonner de la matière à la peau, retrouver les formes pleines d'un visage jeune, redensifier, raviver l'éclat de la peau, augmenter la radiance.

La présente description a aussi pour objet l'utilisation du kombucha et l'utilisation des compositions cosmétiques et dermopharmaceutiques le contenant, seul ou en association, comme ou pour la fabrication de compositions cosmétiques ou dermopharmaceutiques pour traiter en particulier les rides et/ou les ridules, le relâchement cutané et/ou sous cutané de la peau du visage, des hanches, des cuisses, l'avachissement (ou l'effondrement) du micro-relief cutané, la peau flasque, la peau terne.

La présente description concerne également l'utilisation du kombucha, seul ou incorporé dans des compositions cosmétiques ou dermopharmaceutiques pour la préparation de médicaments destinés aux soins de la peau, des muqueuses et des phanères, particulièrement pour lutter contre les signes du vieillissement endogène et/ou exogène.

A titre d'illustration, suivent des exemples, non limitatifs, de la mise en oeuvre de la présente invention.

### Exemple n°1 : Fabrication du kombucha

Dans un récipient de verre à large ouverture, laisser fermenter du thé sucré, contenant au moins 5 g de thé (noir par exemple) par litre et au moins 70 g de sucre par litre, avec le symbiote, par exemple le « champignon de longue vie à kombucha » disponible auprès de la société R. FRANK (à Birkenfeld en Allemagne), dont on ajoute un morceau d'environ 25 à 100 g/l au thé sucré. Placer le récipient de fermentation dans un endroit chaud pendant plusieurs (5 à 12) jours. Après filtration le kombucha est prêt, et le « champignon » est récupéré pour la fermentation suivante. Il est possible dans la fabrication du kombucha d'ajouter dans le mélange de thé sucré et du champignon, 10% de kombucha obtenu lors d'une fabrication précédente, afin d'initialiser plus facilement la fermentation.

| **Exemple n°2** : Crème de jour | | g/100 g |
|---|---|---|
| | Volpo S20 | 2,4 |
| | Volpo S2 | 2,6 |
| | Prostéaryl 15 | 8,0 |
| | Cire d'abeille | 0,5 |
| | Stéaroxy diméthicone | 3,0 |
| | Propylène glycol | 3,0 |
| | Carbomère | 0,25 |
| | Triéthanolamine | 0,25 |
| | kombucha | 3,0 |
| | Eau, conservateurs, parfum | qsp 100 g |

Cette émulsion est utilisée pour illuminer et redensifier la peau du visage.

| **Exemple n°3 :** Gel | | g/100 g |
|---|---|---|
| | Carbomer | 0,3 |
| | Propylène glycol | 2,0 |
| | Glycérine | 1,0 |
| | Vaseline blanche | 1,5 |
| | Cylomethicone | 6,0 |
| | Crodacol C90 | 0,5 |
| | Lubrajel^{R} MS | 10,0 |
| | Triéthanolamine | 0,3 |
| | kombucha | 10,0 |
| | Eau, conservateurs, parfum | qsp 100 g |

Ce gel obtenu de façon extemporanée, peut être utilisé en application quotidienne sur la peau du visage, en particulier pour augmenter la radiance.

| **Exemple n°4 :** Shampooing | | g/100g |
|---|---|---|
| A | Sorbate de potassium | 0,1 |
| | Eau | qsp 100 g |
| B | Empicol ESB3/M | 30,0 |
| | INCRONAM 30 | 4,0 |
| | CROTHIX Liquid | 2,0 |
| | Phenova | 0,8 |
| C | Sodium Hydroxide | 0,1 |
| | Eau | 1,0 |
| D | kombucha | 0,5 |

| **Exemple n°5** : Spray capillaire | | g/ 100g |
|---|---|---|
| A | Eau | qsp 100 g |
| | Ethanol | 10,0 |
| | Sorbate de Potassium | 0,1 |
| B | Procetyl AWS | 0,6 |
| | Nipagine | 0,2 |
| | Butylène Glycol | 3,0 |
| C | kombucha | 3,0 |
| D | parfum | 0,1 |

Méthode : Peser A. Peser B et chauffer à 70°C jusqu'à dissolution. Ajouter B à A, agiter. Ajouter C et D à 40°C. Homogénéiser.

### Exemple n°6 : Efficacité anti-glycation

*Principe :* Un système modèle in vitro, constitué par de la sérum albumine incubée en présence de fructose est incubé pendant 3 semaines. La glycation est observée par la formation de composés fluorescents, généralement des pentosides ou des FFI (furoyl-furanyl-imidazoles).
*Protocole :* La glycation non enzymatique entre sérum albumine et fructose est une réaction spontanée et lente, elle peut être accélérée par la température.
Dans notre test, sérum albumine (à 2%) et fructose à 100 mM sont incubés en milieu tampon phosphate pH 7,4 à 50°C pendant 1 semaine. Les produits finaux de réarrangement après glycation, ici les FFI, présentent une fluorescence naturelle que l'on quantifie (λ ex = 360 nm et λ em = 460 nm). La valeur référence (Témoin) de la glycation après 1 semaine est établie sur l'incubat sérum albumine et fructose. Le témoin positif d'inhibition de la glycation est obtenu par incubation en présence d'aminoguanidine à 0,03%, et les incubations tests sont réalisées en présence de kombucha à différentes concentrations.
*Résultats :* Pour chaque valeur de fluorescence mesurée, le pourcentage de variation par rapport au Témoin est calculé. Les résultats reportés dans le tableau suivant sont des valeurs moyennes sur n = 4 essais.

**Tableau 1 : Inhibition de production des AGE (composés FFI) en présence de kombucha.**

| | Diminution des AGE % par rapport au Témoin |
|---|---|
| Aminoguanidine 0.03% | 89 +/- 5 |
| kombucha 1 % | 71 +/- 7 |
| kombucha 3% | 79 +/- 2 |

Ces résultats indiquent une excellente inhibition de la glycation par l'aminoguanidine, conforme à ce qui est rapporté dans la littérature [BROWNLEE, 1986].
En ce qui concerne le kombucha, on observe aussi une très forte activité anti-glycation, sans effet dose notable, avec une diminution respectivement de - 71 % et - 79% des AGE formés en présence de kombucha à 1 et 3%.

### Exemple n° 7 : Stimulation de différentiation des adipocytes

*Principe :* Les fibroblastes 3T3 L1, en culture in vitro, ont la particularité de se différencier sous l'action d'un cocktail de substances (messagers hormonaux), en pré-adipocytes puis en adipocytes chargés de lipides.
La culture se déroule en 3 étapes : une première étape de multiplication cellulaire jusqu'à confluence, une deuxième étape après ajout du cocktail de différentiation pour l'obtention de pré-adipocytes initiaux (72 heures), et enfin la troisième étape de différentiation active avec stimulation de la lipogenèse (72 heures) : le stockage apparent en gouttelettes lipidiques est alors nettement visible au microscope.
L'enzyme G3PDH (Glycérol-3-Phosphodéshydrogénase), indispensable à la synthèse de triglycérides est exprimée très fortement pendant cette étape active de stockage lipidique.
Le produit à tester est ajouté au début de la deuxième étape en même temps que le cocktail de différentiation.
A l'issue de la période d'incubation on compare l'activité G3PDH entre les pré-adipocytes témoins et ceux incubés en présence du produit testé.
On attend pour un produit favorisant la différentiation, une augmentation de l'activité G3PDH.
*Protocole :* A l'issue de la différentiation provoquée (72 heures), les milieux sont renouvelés sans le cocktail différentiateur mais avec le kombucha à différentes concentrations. Les pré-adipocytes restent donc au contact du kombucha pendant 6 jours.
En fin d'incubation, les cellules sont prélevées et lysées et le test d'activité enzymatique est effectué sur le contenu intracellulaire.
L'activité G3PDH est mesurée par disparition du NADH (λ = 340 nm).
*Résultats :* Les résultats présentés tableau 2 sont des valeurs moyennes établies sur n = 4 essais réalisés, et les valeurs de l'activité enzymatiques sont normalisées au nombre de cellules.

**Tableau 2 : Stimulation de différentiation des adipocytes en culture : augmentation de l'activité G3PDH des préadipocytes traités par le kombucha à différentes concentrations.**

| | Activité G3PDH / 106 cellules % par rapport au contrôle |
|---|---|
| kombucha 0,3% | 21 +/-17 |
| kombucha 1 % | 57.5 +/- 17.7 |
| kombucha 3% | 136 +/- 26 |

Le kombucha augmente l'activité G3PDH de manière dose dépendante.
L'augmentation est très importante et atteint 136% lorsque le kombucha est utilisé à 3%. L'augmentation d'activité de la G3PDH traduit le pouvoir stimulant du kombucha sur la différentiation adipocytaire.
*Observation morphologique sur les pré-adipocytes :* La morphologie des cellules au microscope, à la fin de la période d'incubation en présence de kombucha, montre une population adipocytaire plus riche et différenciée avec inclusions lipidiques par rapport aux cellules témoins.

## Revendications

1. Utilisation cosmétique du kombucha pour lutter contre le vieillissement de la peau, en particulier pour prévenir et inhiber la glycation des protéines, en particulier du collagène et pour stimuler la synthèse des lipides sous-cutanés.

2. Utilisation selon la revendication 1, pour soigner la peau, les muqueuses, et les phanères, notamment pour prévenir les signes du vieillissement endogène et/ou exogène, et en particulier redonner souplesse et élasticité à la peau, améliorer son aspect et la sensation de confort, effectuer un « lipofilling » cosmétique, raffermir la peau du visage, des hanches, des cuisses, tonifier la texture de la peau, redonner de la matière à la peau, redonner les formes pleines d'un visage jeune, redensifier, raviver l'éclat de la peau, augmenter la radiance.

3. Utilisation selon la revendication 1 ou 2 pour traiter les rides et/ou des ridules, le relâchement cutané et/ou sous cutané de la peau du visage, des hanches, des cuisses, l'avachissement (ou l'effondrement) du micro-relief cutané, de la peau flasque, de la peau terne.

4. Utilisation selon l'une des revendications 1 à 3 **caractérisée en ce que** le kombucha est sous une forme liée ou incorporée ou absorbée ou adsorbée à de macro-, micro- et nanoparticules, de macro-, micro- et nanocapsules, dans les textiles, fibres synthétiques ou naturelles, laines et tous matériaux susceptibles d'être utilisés pour réaliser des vêtements et sous-vêtements de jour ou de nuit, directement au contact de la peau ou des cheveux pour en permettre une délivrance topique continue.

5. Utilisation selon l'une quelconque des revendications 1 à 4 **caractérisée en ce que** le kombucha est utilisé sous forme de solution, de dispersion, d'émulsion, de pâte ou de poudre, individuellement ou en pré-mélange ou est véhiculé individuellement ou en pré-mélange par des vecteurs comme les macro-, micro- ou nanocapsules, des liposomes ou des chylomicrons, des macro-, micro- ou nanoparticules, ou les microéponges, ou adsorbé sur des polymères organiques poudreux, les talcs, bentonites et autres supports minéraux.

## Claims

1. Cosmetic use of kombucha to fight against skin ageing, in particular to prevent or inhibit the protein glycation, especially collagen, and to stimulate subcutaneous lipid synthesis.

2. Use according to claim 1, to treat skin, mucosae and appendages, in particular for preventing the signs of endogenous and/or exogenous ageing, and particularly to restore suppleness and elasticity to the skin, to improve its appearance and the feeling of comfort, to carry out a cosmetic "lipofilling", to firm the skin of the face, hips, thighs, to tone the skin texture, to give back matter to the skin, to recover the full forms of a young face, re-densify the volume of the skin, to restore the brightness of the complexion, to increase radiance.

3. Use according to claim 1 or 2, to treat the wrinkles and/or the fine lines, the cutaneous and/or under-cutaneous sagging of the skin of the face, of the hips, of the thighs, the limpness (or the collapse) of cutaneous microrelief, the flask skin, the dull skin.

4. Use according to any of the claims 1 to 3, wherein kombucha is in a form bound to or incorporated in or absorbed in or adsorbed on macro-, micro-, and nanoparticles, or macro-, micro-, and nanocapsules, for the treatment of textiles, natural or synthetic fibres, wools, and any materials that may be used for clothing or for day or night underwear intended to come into contact with the skin or the hair to permit continuous topical delivery.

5. Use according to any of the claims 1 to 4, wherein kombucha is used in the form of solution, dispersion, emulsion, paste, or powder, individually or in pre-mix, or vehiculated individually or as a pre-mix by carriers such as macro-, micro-, or nanocapsules, liposomes or chylomicrons, macro-, micro-, or nanoparticles or macro-, micro-, or nanosponges, or adsorbed on organic polymer powders, talcs, bentonites, or other inorganic.

## Patentansprüche

1. Kosmetische Verwendung von Kombucha zur Bekämpfung der Hautalterung, insbesondere zur Vorbeugung und Hemmung der Glykierung der Proteine, insbesondere des Kollagens, und zur Stimulation der Synthese der subkutanen Fette.

2. Verwendung nach Anspruch 1, zur Pflege der Haut, der Schleimhäute und der Hautanhangsgebilde, insbesondere zur Vorbeugung der Zeichen der endogenen und/oder exogenen Alterung, und insbesondere um der Haut Geschmeidigkeit und Elastizität zurückzugeben, ihr Aussehen und das Behaglichkeitsgefühl zu verbessern, ein kosmetisches "Lipofilling" vorzunehmen, die Haut des Gesichts, der Hüften, der Oberschenkel zu straffen, die Struktur der Haut zu kräftigen, der Haut Substanz zurückzugeben, die vollen Formen eines jungen Gesichts zurückzugeben, wieder zu verdichten, den Glanz der Haut aufzufrischen, das Strahlen zu verstärken.

3. Verwendung nach Anspruch 1 oder 2, zur Behandlung von Falten und/oder Fältchen, der kutanen und/oder subkutanen Erschlaffung der Haut des Gesichts, der Hüften, der Oberschenkel, der Erschlaffung (oder des Einfallens) des Mikroreliefs der Haut, von schlaffer Haut, von matter Haut.

4. Verwendung nach einem der Ansprüche 1 bis 3, **dadurch gekennzeichnet, dass** Kombucha in einer in/an Makro-, Mikro- und Nanopartikeln, Makro-, Mikro- und Nanokapseln gebundenen oder eingebetteten oder absorbierten oder adsorbierten Form, in Textilien, Synthetikoder Naturfasern, Wolle und allen Materialien vorliegt, die geeignet sind, für die Herstellung von Bekleidung und Unterbekleidung für Tag oder Nacht, die direkt mit der Haut oder den Haaren in Kontakt sind, verwendet zu werden, um eine kontinuierliche topische Abgabe hiervon zu ermöglichen.

5. Verwendung nach einem der Ansprüche 1 bis 4, **dadurch gekennzeichnet, dass** Kombucha in Form einer Lösung, einer Dispersion, einer Emulsion, einer Paste oder eines Pulvers, einzeln oder vorgemischt verwendet wird oder durch Vektoren, wie Makro-, Mikro- oder Nanokapseln, Liposome oder Chylomikrone, Makro-, Mikro- oder Nanopartikeln oder Mikroschwämme einzeln oder vorgemischt transportiert wird oder an pulverigen organischen Polymeren, Talken, Bentoniten und anderen mineralischen Trägern adsorbiert wird.
